# EUROPEAN PATENT SPECIFICATION

(11) **EP 2 159 214 B1**
(45) Date of publication and mention of the grant of the patent: **03.04.2013**
(21) Application number: 09169015.6
(22) Date of filing: 31.08.2009
(51) Int. Cl.: C07C 69/54, C08K 5/105

(54) **Amorphous compound and stabilizer for polymers containing the amorphous compound**
Amorphe Verbindung und diese enthaltender Stabilisator für Polymere
Composé amorphe et stabilisateur pour des polymères contenant le composé amorphe

(30) Priority: 29.08.2008 JP 2008221626; 06.04.2009 JP 2009091870; 06.04.2009 JP 2009091871
(43) Date of publication of application: 03.03.2010
(73) Proprietor: Sumitomo Chemical Company, Limited, Tokyo 104-8260 (JP)
(72) Inventor: Kimura, Kenji, Chiba (JP); Kitamura, Kazuhiro, Osaka (JP)
(74) Representative: Vossius & Partner

(56) References cited:
- EP-A1- 0 144 477
- EP-A1- 0 322 166
- EP-A2- 0 500 323

## Description

The present application is filed, claiming the Paris Convention priorities of Japanese Patent Application Nos. 2008-221626 (filed on August 29, 2008), 2009-091870 (filed on April 6, 2009), and 2009-091871 (filed on April 6, 2009).

The invention relates to an amorphous compound and a stabilizer for polymers containing the amorphous compound.

A polymer stabilizer is for providing polymers such as thermoplastic polymers (e.g. polybutadiene) with stability to heat, light, and oxygen and is used by being contained in polymers. As an efficacious component of the stabilizer for polymers, there has been known a compound defined by the following formula and it has also been known well that the compound can be obtained as a powder-like crystal (see, for example, Japanese Patent Application Laid-Open (JP-A) No. 10-273494,).

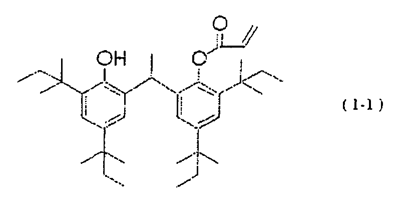

After being dissolved in a hydrocarbon solvent, a stabilizer for polymers containing the above-mentioned compound as an efficacious component is blended as a dissolved material with a polymer to produce a polymer composition.

Further polymer stabilizers art disclosed in EP 0 322 166 A1, EP 0 500 323 A2 and EP 0 144 477 A1.

Since the stabilizer for polymers containing the above-mentioned compounds as an efficacious component are a fine powder, scattering of the powder may occur "at the time of handling the stabilizer for polymers," for example, a step of preparing "a dissolved material consisting of the stabilizer for polymers and a hydrocarbon solvent" to be used for producing a polymer composition and therefore, a stabilizer for polymers with suppressed occurrence of powder-scattering has been desired.

In view of the above state of the art, inventors of the invention have made various investigations and have completed the present invention.

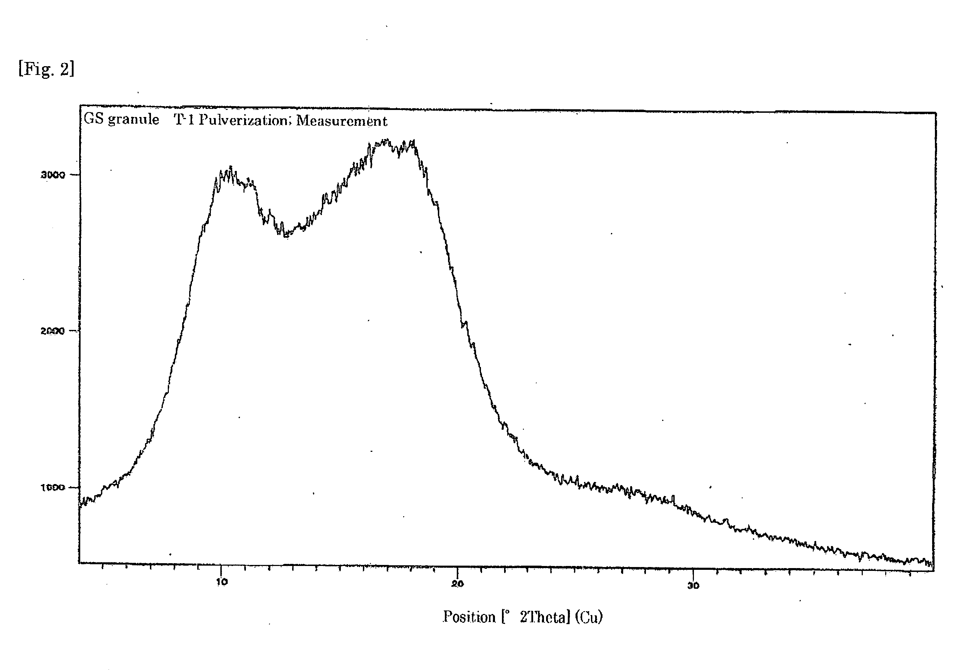

That is, the invention provides the following [1] to [9].
[1] An amorphous compound (hereinafter, referred to also as "the present amorphous compound") defined by the following formula (1): 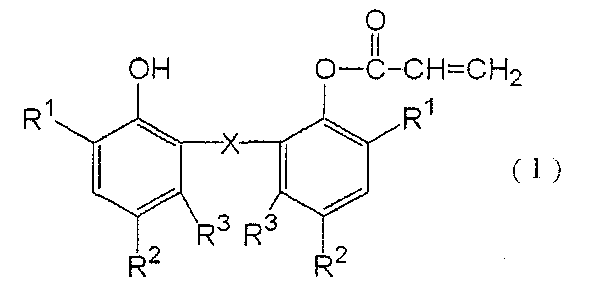 wherein R¹ and R² independently denote a hydrogen atom, an alkyl group with 1 to 8 carbon atoms, or a cycloalkyl group with 5 to 8 carbon atoms; R³ independently denotes a hydrogen atom or an alkyl group with 1 to 8 carbon atoms; X denotes a single bond, a sulfur atom, an oxygen atom, an alkylidene group with 1 to 8 carbon atoms, or a cycloalkylidene group with 5 to 8 carbon atoms: having an endothermic peak at 15 to 25°C determined by differential scanning calorimetry (DSC) and a solution rate of 5 mg/sec or higher in cyclohexane at 25°C in the following test system:
   in the test system for solution rate,
   the measurement is carried out by loading a container (capacity: 100 ml, outer diameter: 55 mm, height: 70 mm) containing 50 g of cyclohexane at 25°C with 3 g of a test substance; rotating fan type stirring blades with 38 mm diameter at a rotation speed of 100 rpm; and measuring the time taken for the test substance to be dissolved.
[2] The amorphous compound described in [1], wherein in the formula (1), R¹ and R² are each a tert-pentyl group; R³ is a hydrogen atom; and X is an ethylidene group.
[3] The amorphous compound described in [2], being in an atomic arrangement state as shown in Fig.2 of the X-ray diffraction pattern determined by X-ray diffractometry using a Cubα spectrum.
[4] The amorphous compound described in [1], wherein in the formula (1), R¹ is a tert-butyl group; R² is a methyl group; R³ is a hydrogen atom; and X is a methylene group.
[5] A method for producing the amorphous compound described in any one of [1] to [4], including a first step of melting a crystalline substance having a melting point of 70°C to 220°C and defined by the following formula (1): 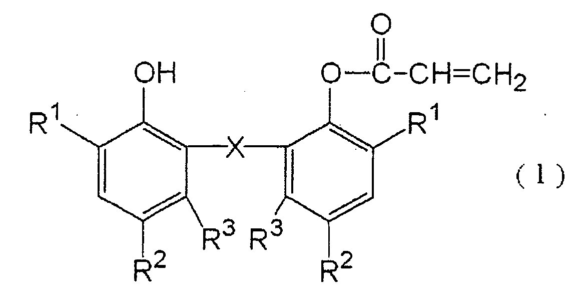 wherein R¹ and R² independently denote a hydrogen atom, an alkyl group with 1 to 8 carbon atoms, or a cycloalkyl group with 5 to 8 carbon atoms; R³ denotes a hydrogen atom or an alkyl group with 1 to 8 carbon atoms; X denotes a single bond, a sulfur atom, an oxygen atom, an alkylidene group with 1 to 8 carbon atoms, or a cycloalkylidene group with 5 to 8 carbon atoms by heating at a temperature equal to or higher than the melting point and a second step of cooling and solidifying the melted substance obtained in the first step.
[6] A stabilizer for polymers, containing the amorphous compound described in any one of [1] to [4].
[7] The stabilizer for polymers described in [6], having a particulate shape.
[8] A process for producing a polymer composition, including a first step of dissolving the stabilizer for polymers described in [6] or [7] in a hydrocarbon solvent and a second step of blending the dissolved material obtained in the first step with a polymer.
[9] The process described in [8], wherein the polymer is a thermoplastic polymer.

The amorphous compound of the invention has an excellent "capability of quickly dissolving in a hydrocarbon solvent" and the stabilizer for polymers of the invention does not cause scattering of powder and is thus excellent as a stabilizer for polymers.
Fig. 1 is a drawing showing an endothermic peak in differential scanning calorimetry using DSC for the present amorphous compound.
Since the peak is a broad peak in the phase transition, the drawing shows that the amorphous compound is in an amorphous atomic arrangement state.
Fig. 2 is a drawing showing an X-ray diffraction pattern by X-ray diffraction measurement using a CuKα spectrum for the present amorphous compound. Since the pattern contains broad peaks, the drawing shows that the amorphous compound is in an amorphous atomic arrangement state.
Fig. 3 is a drawing showing an endothermic peak in differential scanning calorimetry using DSC for a conventional crystalline compound. Since the peak is a sharp peak at a single point, that is, the melting point, the drawing shows that the compound is in a crystalline atomic arrangement state.
Fig. 4 is a drawing showing an X-ray diffraction pattern by X-ray diffraction measurement using a CuKα spectrum for a conventional crystalline compound. Since the pattern contains sharp diffraction peaks of the respective crystal lattice planes, the drawing shows that the compound is in a crystalline atomic arrangement state.
The amorphous compound of the invention is an amorphous compound defined by the following formula (1): 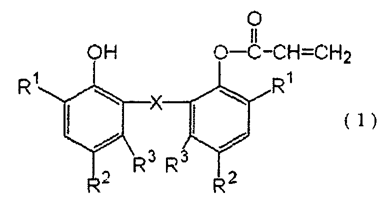 wherein R¹ and R² independently denote a hydrogen atom, an alkyl group with 1 to 8 carbon atoms, or a cycloalkyl group with 5 to 8 carbon atoms; R³ independently denotes a hydrogen atom or an alkyl group with 1 to 8 carbon atoms; X denotes a single bond, a sulfur atom, an oxygen atom, an alkylidene group with 1 to 8 carbon atoms, or a cycloalkylidene group with 5 to 8 carbon atoms: having an endothermic peak at 15 to 25°C determined by differential scanning calorimetry (DSC) and a solution rate of 5 mg/sec or higher in cyclohexane at 25°C in the following test system:
in the test system for solution rate,
the measurement is carried out by loading a container (capacity: 100 ml, outer diameter: 55 mm, height: 70 mm) containing 50 g of cyclohexane at 25°C with 3 g of a test substance; rotating fan type stirring blades with 38 mm diameter at a rotation speed of 100 rpm; and measuring the time taken for the test substance to be dissolved.

In the formula (1), R¹ and R² independently denote a hydrogen atom, an alkyl group with 1 to 8 carbon atoms, or a cycloalkyl group with 5 to 8 carbon atoms. Herein, examples of the alkyl group include a methyl group, an ethyl, an n-propyl group, an isopropyl group, an n-butyl group, an isobutyl group, a tert-butyl group, an n-pentyl group, an isopentyl group, a tert-pentyl group, a 2-ethylhexyl group, and the like. Further, examples of the cycloalkyl group include a cyclopentyl group, a cyclohexyl group, a cyclooctyl group, a 3-methylcyclopentyl group, a 4-methylcyclopentyl group, a 3-methylcyclohexyl group, and the like. Especially, for example, a methyl group, a tert-butyl group, or a tert-pentyl group is preferably exemplified.

R³ independently denotes a hydrogen atom or an alkyl group with 1 to 8 carbon atoms. Examples of the alkyl group for R³ include alkyl groups exemplifies for R¹. Especially, a hydrogen atom or a methyl group is preferably exemplified.

X denotes a single bond, a sulfur atom, an oxygen atom, an alkylidene group with 1 to 8 carbon atoms, or a cycloalkylidene group with 5 to 8 carbon atoms.

Herein, examples of the alkylidene group include a methylene group, an ethylidene group, a propylidene group, a butylidene group, and the like. Further, examples of the cycloalkylidene group include a cyclopentylidene group, a cyclohexylidene group, and the like. Especially, a methylene group, an ethylidene group, or a butylidene group is preferably exemplified.

Specific examples of the present amorphous compound include
2-[1-(2-hydroxy-3,5-di-tert-pentylphenyl)ethyl]-4,6-di-tert-pentylphenyl acrylate,
2,4-di-tert-butyl-6-[1-(3,5-di-tert-butyl-2-hydroxyphenyl)ethyl]phenyl acrylate,
2-tert-butyl-6-(3-tert-butyl-2-hydroxy-5-methylbenzyl)-4-methylphenyl acrylate,
2-tert-butyl-6-[1-(3-tert-butyl-2-hydroxy-5-methylphenyl)ethyl]-4-methylphe nyl acrylate,
2-tert-butyl-6-[1-(3-tert-butyl-2-hydroxy-5-methylphenyl)propyl]-4-methylph enyl acrylate,
2-tert-butyl-6-[1-(3-tert-butyl-2-hydroxy-5-propylphenyl)ethyl]-4-propylphen yl acrylate, and
2-tert-butyl-6-[1-(3-tert-butyl-2-hydroxy-5-isopropylphenyl)ethyl]-4-isopropy lphenyl acrylate, and the like.

Specific and preferable examples of the present amorphous compound include 2-[1-(2-hydroxy-3,5-di-tert-pentylphenyl)ethyl]-4,6-di-tert-pentylphenyl acrylate, that is, a substance defined by the formula (1) in which R¹ and R² are each a tert-pentyl group; R³ is a hydrogen atom; and X is an ethylidene group and 2-tert-butyl-6-(3-tert-butyl-2-hydroxy-5-methylbenzyl)-4-methylphenyl acrylate, that is, a substance defined by the formula (1) in which R¹ is a tert-butyl group; R² is a methyl group; R³ is a hydrogen atom; and X is a methylene group.

The present amorphous compound has an endothermic peak at 15 to 25°C determined by differential scanning calorimetry (DSC).

Further, the present amorphous compound has a solution rate of 5 mg/sec or higher, preferably 5 mg/sec or higher and 9 mg/sec or lower in "a test system for solution rate" measured by loading a container (capacity: 100 ml, outer diameter: 55 mm, height: 70 mm) containing 50 g of cyclohexane at 25°C with 3 g of a test substance and rotating fan type stirring blades with 38 mm diameter at a rotation speed of 100 rpm, and measuring the time taken for the test substance to be dissolved.

As the present amorphous compound, there is exemplified an amorphous compound which is in an atomic arrangement state as shown in Fig.2 of the X-ray diffraction pattern determined by X-ray diffractometry using a CuKα spectrum.
Fig. 2 shows the results of the X-ray diffractometry showing the peak intensity in the axis of ordinate and the angle 2θ of the diffraction line in the axis of abscissa. Preferable examples of the present amorphous compound include substances of amorphous compounds having diffraction patterns with two broad peaks whose peak tops exist in 10 to 12° and 16 to 19° of 2θ. More preferable examples include amorphous compounds having diffraction patterns with two broad peaks whose peak tops exist in 10 to 11° and 16 to 18° of 2θ.

The shape of the present amorphous compound may be plate-like, fine powder-like, pellet-like, granular, tablet-like, approximately spherical, approximately semi-spherical, flaky, etc. and is not particularly limited; however, approximately spherical and approximately semi-spherical shapes are preferable. Further, the size of the present amorphous compound is not particularly limited; however, its particle diameter may be, for example, about 1 mm to about 4 mm. A preferable particle diameter may be, for example, about 2 mm to about 4 mm. The height of the present amorphous compound may be, for example, about 1 mm to about 4 mm. A preferable height may be, for example, about 1 mm to about 3 mm. The weight per one particle of the present amorphous compound is not particularly limited; however, it may be, for example, about 6 mg to about 12 mg. Moreover, the hardness of the present amorphous compound is not particularly limited; however, it may be, for example, about 10 N to about 30 N.

In addition, in the case the shape of the present amorphous compound is plate-like, as necessary, the present amorphous compound may be pulverized to be flaky. As a production process of the present amorphous compound, there is exemplified the process, including a first step of melting a crystalline substance having a melting point of 70°C to 220°C, preferably 100 to 140°C and defined by the following formula (1): 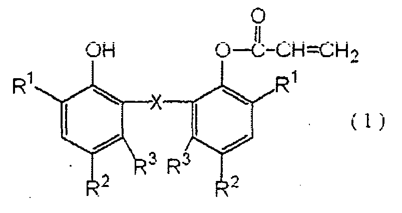 wherein R¹ and R² independently denote a hydrogen atom, an alkyl group with 1 to 8 carbon atoms, or a cycloalkyl group with 5 to 8 carbon atoms; R³ independently denotes a hydrogen atom or an alkyl group with 1 to 8 carbon atoms; X denotes a single bond, a sulfur atom, an oxygen atom, an alkylidene group with 1 to 8 carbon atoms, or a cycloalkylidene group with 5 to 8 carbon atoms by heating at a temperature equal to or higher than the melting point and a second step of cooling and solidifying the melted substance obtained in the first step.

In the first step of the process of the present amorphous compound, the above-mentioned crystalline substance may be melted by heating at a temperature equal to or higher than the melting point and the "temperature equal to or higher than the melting point" may be, for example, about 90°C to about 250°C and preferably about 120°C to about 160°C.

In the second step of the process of the present amorphous compound, the melted substance obtained in the first step may be cooled and solidified, and the temperature and time for "cooling" may be, for example, about 50°C or lower for about 10 sec. or longer. Particularly, they are preferably about 0°C to about 50°C for about 10 sec. or longer and about 2 min. or shorter and more preferably about 0°C to about 40°C for about 20 sec. to about 2 min.

As the second step of cooling and solidifying the melted substance obtained in the first step, there are exemplified a process of spraying or dripping the melted substance obtained in the first step to a cooled heat exchange plate (e.g. a sheet or the like made of a metal such as stainless steel); a process of dripping the melted substance obtained in the first step to cooled water or a poor solvent; and a process of continuously extruding the melted substance obtained in the first step on a cooled belt.

The process for dripping the melted substance may be, for example a process of dripping from a dropping tube and specifically, a process of dripping the melted substance after it is packed in a roll drop type granulation apparatus, the Rotoform^{®} granulation apparatus, or the like.

Herein, the roll drop type granulation apparatus is generally a granulation apparatus provided with a rotary drum having projections and having a mechanism of scraping the melted substance with the tip end parts of the projections and dripping the melted substance on a heat exchange plate by the function of centrifugal force and/or gravity obtained by rotating the rotary drum. The Rotoform^{®} granulation apparatus is a granulation apparatus generally having a cylindrical part which has holes and a structure of receiving the melted substance in the inside of the cylindrical part and thus having a mechanism of dripping the melted substance on a heat exchange plate through the holes. Dripping using the Rotoform^{®} granulation apparatus is particularly preferred.

To control the weight per one particles of the present amorphous compound to be a desired value, in the case of a process of dripping the melted substance from a dropping tube, the diameter of the dropping tube or the viscosity of the melted substance may be adjusted so as to control the dripping amount of the melted substance from the dropping tube. Specifically, for example, in the case of the roll drop type granulation apparatus, the amount of the melted substance to be scraped by the tip end parts of the projections may be controlled and in the case of the Rotoform^{®} granulation apparatus, the size of the holes and the viscosity of the melted substance may be adjusted to control the dripping amount of the melted substance from the dropping tube.

As the "cooled heat exchange plate," there is exemplified a heat exchange plate heated to about 0°C to about 50°C. Specific examples thereof include a belt made of stainless steel adjusted to a prescribed temperature by water or the like, a belt made of stainless steel adjusted to a prescribed temperature by cold blow or the like, a stainless plate adjusted to a prescribed temperature by water or the like, and a stainless plate adjusted to a prescribed temperature by cold blow or the like. In addition, the face of the heat exchange plate to which the melted substance is dripped is better to be smooth.

A stabilizer for polymers of the invention is characterized in that the stabilizer contains the above-mentioned amorphous compound.

The content of the amorphous compound in the stabilizer for polymers of the invention is, for example, about 1% by weight or higher, preferably about 75% by weight or higher, more preferably about 85% by weight, and even more preferably about 95% by weight or higher in the entire weight of the stabilizer for polymers of the invention. Of course, the stabilizer for polymers of the invention may be made of the amorphous compound (that is, the case where the amorphous compound accounts for 100% by weight in the entire weight of the stabilizer for polymers of the invention).

The stabilizer for polymers of the invention may contain various kinds of additives to an extant that the effects of the invention are not inhibited. Examples of such additives include phenolic antioxidants such as n-octadecyl-3-(4-hydroxy-3,5-di-tert-butylphenyl) propionate (melting point 50 to 55°C), 2,6-di-tert-butyl-4-methyl phenol (melting point 69°C (freezing point)),
2,2-thio-diethylene-bis-[3-(3,5-di-tert-butyl-4-hydroxyphenyl)propionate] (melting point 63°C or higher), tri-ethylene
glycol-bis-[3-(3-tert-butyl-5-methyl-hydroxyphenyl)propionate] (melting point 76 to 79°C),
3.9-bis-[2-{3-(3-tert-butyl-4-hydroxy-5-methylphenyl)propionyloxy}-1,1-dimethylethyl]-2,4,8,10-tetraoxaspiro[5.5]undecane (melting point 110 to 130°C),
tetrakis{3-(3,5-di-tert-butyl-4-hydroxyphenyl)-propionic acid}pentaerythrityl ester (melting point 110 to 130°C),
2-tert-butyl-6-(3-tert-butyl-2-hydroxy-5-methylbenzyl)-4-methylphenyl acrylate (melting point 130°C or higher),
2-[1-(2-hydxoxy-3,5-di-tert-pentylphenyl)ethyl]-4,6-di-tert-pentylphenyl acrylate (melting point 119°C),
1,3,5-trimethyl-2,4,6-tris(3,5-di-tert-butyl-4-hydroxybenzyl)benzene (melting point 240 to 245°C), tris(3,5-di-tert-butyl-4-hydroxybenzyl)isocyanurate (melting point 218 to 223°C),
1,3,5-tris(4-tert-butyl-3-hydroxy-2,6-dimethylbenzyl)-1,3,5-triazine-2,4,6-(1 H, 3H, 5H)-trione (melting point 159 to 162°C),
2,2'-methylenebis(6-tert-butyl-4-methylphenol) (melting point 128°C or higher), 4,4'-butylidenebis(6-tert-butyl-3-methylphenol) (melting point
209°C or higher), and 4,4'-thiobis(6-tert-butyl-3-methylphenol) (melting point 160°C or higher);

sulfur type antioxidants such as 3,3'-thiodipxopionic acid di-n-dodecyl ester (melting point 40 to 42°C), 3,3'-thiodipropionic acid di-n-tetradecyl ester (melting point 49 to 54°C), 3,3'-thiodipropionic acid di-n-octadecyl ester (melting point 65 to 67°C), and tetrakis(3-dodecylthiopropionic acid)pentaerythrityl ester (melting point about 46°C);

phosphorus type antioxidants such as tris(2,4-di-tert-butylphenyl)phosphite (melting point 183 to 187°C), bis(2,4-di-tert-butylphenyl)pentaerythritol diphosphite (melting point 160 to 180°C), bis(2,6-di-tert-butyl-4-methylphenyl)pentaerythritol diphosphite (melting point 237 to 238°C), bis(2,4-di-cumylphenyl)pentaerythritol diphosphite (melting point 221 to 230°C), tetrakis(2,4-di-tert-butylphenyl)-4,4'-biphenylene diphosphonite (melting point 75 to 90°C), and bis-[2,4-di-tert-butyl-(6-methyl)phenyl]ethyl phosphite (melting point 89 to 92°C); hindered amine type antioxidants such as sebacic acid bis(2,2,6,6-tetramethyl-4-piperidyl) ester (melting point 81 to 86°C), 2,2,6,6-tetramethyl-4-piperidyl methacrylate (melting point 58°C), and poly[{6-(1,1,3,3-tetramethylbutyl)amino-1,3,5-triazine-2,4-diyl} {(2,2,6,6-tetramethyl-4-piperidyl)imino}-1,6-hexmethylene {(2,2,6,6-tetramethyl-4-piperidyl)imino} (melting point 100 to 135°C);

ultraviolet absorbents such as 2-hydroxy-4-n-octyloxybenzophenone (melting point 45°C or higher),
2-(2H-benzotriazol-2-yl)-4,6-di-tert-pentylphenol (melting point 77°C or higher), 2-[4,6-bis(2,4-dimethylphenyl)-1,3,5-triazin-2-yl-]-5-(octyloxy)phenol (melting point 87 to 89°C), 2-(2-hydroxy-5-methylphenyl)benzotriazole (melting point 127°C),

2-(3-tert-butyl-2-hydroxy-5-methylphenyl)-5-chlorobenzotriazole (melting point 137°C), and 2,4-di-tert-butylphenyl 3,5-di-tert-butyl-4-hydroxybenzoate (melting point 192°C);

nucleating agents such as an α-naphthalenesulfonic acid Na salt, an α -naphthalenesulfonic acid Mg salt, an α-naphthalenesulfonic acid Ca salt, an α-naphthalellesulfonic acid Al salt, an 8-aminonaphthalenesulfonic acid Na salt, a benzenesulfonic acid Na salt, a benzenesulfonic acid Mg salt, a benzenesulfonic acid Ca salt, a benzenesulfonic acid Al salt, a

2,5-dichlorobenzenesulfonic acid Ca salt, a 2,5-dichlorobenzenesulfonic acid Mg salt, an m-xylenesulfonic acid Ca salt, an m-xylenesulfonic acid Mg salt, benzoic acid (melting point 122°C), p-isopropylbenzoic acid,
o-tert-butylbenzoic acid, p-tert-butylbenzoic acid, monophenylacetic acid (melting point 77°C), diphenylacetic acid, a diphenylacetic acid Li salt, a diphenylacetic acid Na salt, a diphenylacetic acid Mg salt, a diphenylacetic acid Ca salt, a diphenylacetic acid Ba salt, a diphenylacetic acidAl salt, phenyldimethylacetic acid, a phenyldimethylacetic acid Li salt, a phenyldimethylacetic acid Na salt, a phenyldimethylacetic acid Mg salt, a phenyldimethylacetic acid Ca salt, a phenyldimethylacetic acid Ba salt, a phthalic acid Mg salt, succinic acid (melting point 185°C), a succinic acid Li salt, a succinic acid Na salt, a succinic acid Mg salt, a succinic acid Ca salt, a succinic acid Ba salt, glutaric acid (melting point 95 to 99°C), a glutaric acid Li salt, a glutaric acid Na salt, a glutaric acid Mg salt, a glutaric acid Ca salt, a glutaric acid Ba salt, adipic acid (melting point 151 to 153°C), suberic acid, a suberic acid Li salt, a suberic acid Na salt, a suberic acid Mg salt, a suberic acid Ca salt, a suberic acid Ba salt, sebacic acid, a sebacic acid Li salt, a sebacic acid Na salt, a sebacic acid Mg salt, a sebacic acid Ca salt, a sebacic
acid Al salt, diphenylphsophinic acid (melting point 193 to 196°C), a diphenylphosphinic acid Li salt, a diphenylphosphinic acid Na salt, a diphenylphosphinic acid K salt, a diphenylphosphinic acid Ca salt, a diphenylphosphinic acid Mg salt, a diphenylphosphinic acid Al salt, a 4,4-'-dichlorodiphenylphosphinic acid Li salt, a

4,4'-dimethyldiphenylphosphinic acid Na salt, dinaphthylphosphinic acid, a dinaphthylphosphinic acid Li salt, a dinaphthylphosphinic acid Na salt, a dinaphthylphosphinic acid Mg salt, a dinaphthylphosphinic acid Ca salt, and a dinaphthylphosphinic Al salt;

metal soaps including fatty acid metal salts such as calcium stearate and hydrotalcite;

inorganic or organic anti-blocking agents such as aluminum silicate, synthetic silica, natural silica, zeolites, kaolin, and diatomaceous earth;

pigments such as carbon black, titanium oxide, phthalocyanine-based pigments, quinacridone-based pigments,

isoindolinone-based pigments, perylene- or perynine-based pigments, quinophthalone-based pigments, diketopyrrolo-pyrrole-based pigments, dioxazine-based pigments, disazo condensation pigments, and benzimidazolone-based pigments;

flame-retardants such as decabromobiphenyl, antimony trioxide, phosphorus type flame-retardants, and aluminum hydroxide;

anti-static agents such as quaternary ammonium salt type cationic surfactants, betaine type amphoteric surfactants, alkyl phosphate type anionic surfactants, cationic surfactants such as primary amine salts, secondary amine salts, tertiary amine salts, quaternary amine salts, and pyridine derivatives, anionic surfactants such as sulfated oils, soap, sulfated ester oils, sulfated amide oils, sulfated ester salts of olefins, fatty alcohol sulfuric acid ester salts, alkyl sulfuric acid ester salts, fatty acid ethylsulfonic acid salts, alkylnaphthalenesulfonic acid salts, alkylbenzenesulfonic acid salts, succinic acid ester sulfonic acid salts, and phosphoric acid ester salts, nonionic surfactants such as polyhydric alcohols partially esterified with fatty acids, fatty alcohol ethylene oxide adducts, fatty acid ethylene oxide adducts, fatty acid amino or fatty acid amide ethylene oxide adducts, alkylphenol ethylene oxide adducts, ethylene oxide adducts of polyhydric alcohols partially esterified with fatty acids, and polyethylene glycols, and amphoteric surfactants such as carboxylic acid derivatives and imidazoline derivatives;

and also a lubricant, a filler, a plasticizer, a processing aid, a foaming agent, an emulsifier, a brightener, and a binder may be contained in the stabilizer for polymers of the invention.

The contents of the various kinds of additives in the stabilizer for polymers of the invention may be about 25% by weight or less, preferably about 15% by weight or less, and more preferably about 5% by weight or less in the entire weight of the stabilizer for polymers of the invention.

The shape of the present amorphous compound may be, for example, particulate or plate-like and preferably particulate. Specific examples of the particulate shape include pellet-like, granular, tablet-like, approximately spherical, approximately semi-spherical, flaky, and preferably approximately spherical or approximately semi-spherical. Further, the weight per one particle of the particulate amorphous compound of the invention is preferably 1 mg or more of the present amorphous compound and more preferably, for example, 1 mg to about 25 mg. The particle diameter per one particle of the particulate amorphous compound of the invention is, for example, 1 mm to about 6 mm. Especially, it is preferably about 2 mm to about 5 mm. Further, the height is, for example, about 1 mm to about 4 mm. Especially, the height is preferably about 1 mm to about 3 mm. Moreover, the hardness of the particulate amorphous compound of the invention is, for example, about 10 N to about 30 N.

Examples of the production method of the polymer composition containing the present amorphous compound include (1) a production method including a first step of dissolving the stabilizer for polymers of the invention in a hydrocarbon solvent and a second step of blending the dissolved material obtained in the first step with a polymer and (2) a production method including a step of melting and kneading the stabilizer for polymers of the invention with a polymer.

Examples of the hydrocarbon solvent to be used in the first step of the above-mentioned production method (1) include hydrocarbon solvents such as pentane, hexane, cyclopentane, and cyclohexane.

The mixing ratio of the stabilizer for polymers of the invention and the hydrocarbon solvent in the first step is not particularly limited as long the stabilizer for polymers of the invention is dissolved in the hydrocarbon solvent. The mixing ratio may be, for example, 10 to 90% by weight of the stabilizer for polymers of the invention in the total amount of the stabilizer for polymers of the invention and the hydrocarbon solvent.

The blending method is also not particularly limited and an example thereof is a blending method of adding the stabilizer for polymers of the invention to a hydrocarbon solvent stored in a container in a nitrogen atmosphere and stirring the mixture at about -10 to 70°C, and an example thereof is a blending method of adding a hydrocarbon solvent to the stabilizer for polymers of the invention stored in a container in a nitrogen atmosphere and stirring the mixture at about -10 to 70°C.

The blending ratio of the stabilizer for polymers of the invention and a polymer in the second step of the above-mentioned production method (1) and in the step of the production method (2) may be a ratio of blending 2 parts by weight or less of the stabilizer for polymers of the invention to 100 parts by weight of a polymer. The blending ratio is preferably 0.01 parts by weight or higher and 2 parts by weight or lower and more preferably 0.01 1 parts by weight or higher and 1 part by weight or lower.

In the production method of the polymer composition of the invention, the polymer is preferably a thermoplastic polymer.

Herein, the thermoplastic polymer is not particularly limited as long as it is a commercialized resin and examples thereof include polypropylene type resins such as ethylene-propylene copolymers, polyethylene type resins (high density polyethylene (HD-PE), low density polyethylene (LD-PE), linear low density polyethylene (LLDPE), etc.), methylpentene polymers, ethylene-ethyl acrylate copolymers, ethylene-vinyl acetate copolymers, polystyrenes (polystyrenes such as poly(p-methylstyrene) and
poly(α-methylstyrene), acrylonitrile-styrene copolymers,
acrylonitrile-butadiene-styrene copolymers, special acryl
rubber-acrylonitrile-styrene copolymers, acrylonitrile-chlorinated

polyethylene-styrene copolymers, styrene-butadiene copolymers, etc.), polyethylene chlorides, polychloroprene, chlorinated rubber, poly(vinyl chloride), poly(vinylidene chloride), methacrylic resins, ethylene-vinyl alcohol copolymers, fluoro resins, polyacetals, grafted polyphenylene ether resins, polyphenylene sulfide resins, polyurethanes, polyamides, polyester resins (e.g. polyethylene terephthalate, polybutylene terephthalate, etc.), polycarbonates, polyacrylates, polysulfones, polyether ether ketones, polyether sulfones, aromatic polyester resins, diallyl phthalate prepolymers, silicone resins, 1,2-polybutadiene, polyisoprene, butadiene/acrylonitrile copolymers, and ethylene-methyl methacrylate copolymers and particularly,

due to the good molding processability, polyethylene type resins, polypropylene type resins, and polystyrenes are preferable.

Herein, the polypropylene type resins mean polyolefins containing a structural unit derived from propylene and specifically, examples thereof include crystalline propylene homopolymers, propylene-ethylene random copolymers, propylene-α-olefin random copolymers,

propylene-ethylene-α-olefin copolymers, and block copolymers consisting of a propylene homopolymer component or a copolymer component consisting mainly of propylene as well as a copolymer component of propylene with ethylene and/or α-olefins.

In the case a polypropylene type resin is used as the thermoplastic polymer in the invention, one kind of polypropylene type resin may be used or two or more kinds thereof may be blended and used.

The α-olefins may be α-olefins with 4 to 12 carbon atoms and examples thereof include 1-butene, 1-pentene, 1-hexene, 4-methyl-1-pentene, 1-octene, and 1-decene and preferably 1-butene, 1-hexene, and 1-octene.

Examples of the propylene-α-olefin random copolymers include a propylene-1-butene random copolymer, a propylene-1-hexene random copolymer, and a propylene-1-octene random copolymer.

Examples of the propylene-ethylene-α-olefin copolymers include a propylene-ethylene-1-butene copolymer, a propylene-ethylene-1-hexene copolymer, and a propylene-ethylene-1-octene copolymer.

Examples of the copolymer component consisting mainly of propylene, composed of the polypropylene type block copolymers consisting of a propylene homopolymer component or a copolymer component consisting mainly of propylene as well as a copolymer component of propylene with ethylene and/or α-olefins, include a propylene-ethylene copolymer component, a propylene-1-butene copolymer component and a propylene-1-hexene copolymer component and examples of the copolymer component of propylene with ethylene and/or α-olefins include a propylene-ethylene copolymer component, a propylene-ethylene-1-butene copolymer component, a propylene-ethylene-1-hexene copolymer component, a

propylene-ethylene-1-octene copolymer component, a propylene-1-butene copolymer component, a propylene-1-hexene copolymer component, and a propylene-1-actente copolymer component. The content of ethylene and/or α -olefins with 4 to 12 carbon atoms in the copolymer component of propylene with ethylene and/or α-olefins is, for example, 0.01 to 20% by weight.

Further, examples of the polypropylene type block copolymers consisting of a propylene homopolymer component or a copolymer component consisting mainly of propylene as well as a copolymer component of propylene with ethylene and/or α-olefins, include a propylene-ethylene block copolymer, a (propylene)-(propylene-ethylene) block copolymer, a (propylene)-(prapylene-ethylene-1-butene) block copolymer, a (propylene)-(propylene-ethylene-1-hexene) block copolymer, a (propylene)-(propylene-1-butene) block copolymer, a

(propylene)-(propylene-1-hexene) block copolymer, a (propylene-ethylene)-(propylene-ethylene-1-butene) block copolymer, a (propylene-ethylene)-(propylene-ethylene-1-hexene) block copolymer, a (propylene-ethylene)-(propylene-1-butene) block copolymer, a (propylene-ethylene)-(propylene-1-hexene) block copolymer, a

(propylene-1-butene)-(propylene-ethylene) block copolymer, a (propylene-1-butene)-(propylene-ethylene-1-butene) block copolymer, a (propylene-1-butene)-(propylene-ethylene-1-hexene) block copolymer, a (prapylene-1-butene)-(propylene-1-butene) block copolymer, and a (propylene-1-butene)-(propylene-1-hexene) block copolymer.

Further, in the invention, in the case a polypropylene type resin is used as the thermoplastic polymer, crystalline propylene homopolymers and polypropylene type block copolymers consisting of a propylene homopolymer component or a copolymer component consisting of propylene as well as a copolymer component of propylene with ethylene and/or α-olefins with 4 to 12 carbon atoms are preferable. The polypropylene type block copolymers consisting of a propylene homopolymer component or a copolymer component consisting mainly of propylene as well as a copolymer component of propylene with ethylene and/or α-olefins with 4 to 12 carbon atoms are more preferable.

### EXAMPLES

Hereinafter, the invention will be described in more detail with reference to examples and comparative examples.

As a crystalline substance defined by the formula (1) and having a melting point of 100°C to 140°C and in the form of a fine powder with a weight per one particle less than 1 mg/each particle,

2-[1-(2-hydroxy-3,5-di-tert-pentylphenyl)ethyl-4,6-di-tert-pentylphenyl

acrylate (melting point 115°C, manufactured by Sumitomo Chemical Co., Ltd.) (hereinafter, sometimes referred to as a "compound (1)") and

2-tert-butyl-6-(3-tert-butyl-2-bydroxy-5-methylbenzyl)-4-methylphenyl

acrylate (melting point 180°C, manufactured by Sumitomo Chemical Co., Ltd.) (hereinafter, sometimes referred to as a "compound (2)") were used.

### (Example 1)

The compound (1) was put in a melting tank heated to 145°C and melted. Next, after dripped on a stainless plate cooled with cooling water at 30°C, the obtained melted substance was cooled on the stainless plate for 25 second and solidified to obtain an approximately semi-spherical amorphous compound. The size of the obtained amorphous compound was 3.4 mm in particle diameter (width), 2.2 mm in height, and its weight was 8.62 mg/particle, and its hardness was 24.79 N.

Next, the obtained amorphous compound was subjected to the following DSC analysis, XRD analysis, and solution rate measurement.

### (DSC analysis)

Using a differential scanning calorimeter DSC-60A manufactured by Shimadzu Corporation, the obtained amorphous compound was air-tightly closed in an aluminum cell and after the aluminum cell was inserted into a sample holder of the differential scanning calorimeter, while the sample holder being heated to 150°C in a nitrogen atmosphere at a speed of 10°C/min, the endothermic peak was observed.

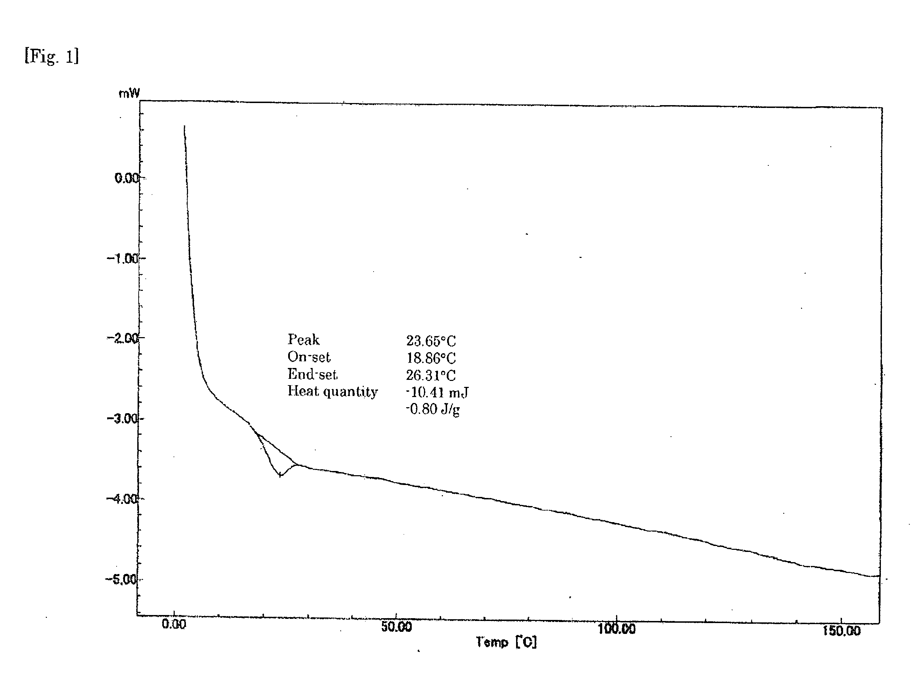

As a result, as shown in Fig. 1, the endothermic peak of the obtained amorphous compound was 23.7°C.

### (XRD analysis)

The obtained amorphous compound was pulverized. Next, after the obtained pulverized material was inserted into a sample holder of RINT 2000 vertical type goniometer manufactured by Rigaku Co., Ltd., the X-ray diffraction pattern was measured using a CuKα spectrum by X-ray diffractometry. The obtained X-ray diffraction pattern is shown in Fig. 2.

As a result, as shown in Fig. 2, since the pattern contains broad peaks, it was confirmed that the amorphous compound was in an amorphous atomic arrangement state.

### (Weight measurement per one particle of particulate amorphous compound)

Using a precision balance manufactured by METTLER TOLEDO, the weight per one particle of the obtained amorphous compound was measured. The measurement was repeated 20 times and the average value was defined as the "weight per one particle of the particulate amorphous compound."

### (Hardness measurement of particulate amorphous compound)

Using a digital force gauge FGP-5 manufactured by SHIMPO, the hardness of the obtained amorphous compound was measured as follows. The measurement was repeated 20 times and the average value was defined as the "hardness of the particulate amorphous compound."

The obtained amorphous compound was set on a sample stand of a measurement apparatus. The tip end of a probe attached to the measurement apparatus was lowered to the position of the amorphous compound set on the sample stand to apply pressure to the amorphous compound. The scale of a crushing pressure meter was read at the time the amorphous compound was crushed and the value was defined as the "hardness of the particulate amorphous compound."

### (Measurement of particle diameter (width) and height of particulate amorphous compound)

Using a slide caliper, the particle diameter (width) and height of the obtained amorphous compound were measured. The measurement was repeated 10 times and the average values were defined as the "particle diameter (width) of the particulate amorphous compound" and the "height of the particulate amorphous compound."

### (Solution rate measurement)

A container (capacity: 100 ml, outer diameter: 55 mm, height: 70 mm) containing 50 g of cyclohexane at 25°C was loaded with 3 g of a test substance; fan type stirring blades with 38 mm diameter were rotated at a rotation speed of 100 rpm; and the time taken for the test substance to be dissolved was measured. The result is shown in Table 1 as the solution rate based on the "mg/sec" unit.

### (Example 2)

A semi-spherical solid amorphous compound was obtained in the same manner as in Example 1, except that "a mixture obtained by mixing the compound (1) and the compound (2) at a weight ratio of 99.5 : 0.5" was used in place of the "compound (1)" used in Example 1. The size of the obtained amorphous compound was 3.1 mm in particle diameter (width), 1.8 mm in height, and its weight was 9.65 mg/particle, and its hardness was 23.77 N.

Next, the obtained amorphous compound was subjected to the above-mentioned DSC analysis, XRD analysis, and solution rate measurement.

### (Example 3)

A semi-spherical solid amorphous compound was obtained in the same manner as in Example 1, except that "a mixture obtained by mixing the compound (1) and the compound (2) at a weight ratio of 95 : 5" was used in place of the "compound (1)" used in Example 1. The size of the obtained amorphous compound was 2.7 mm in particle diameter (width), 1.7 mm in height, and its weight was 9.99 mg/particle, and its hardness was 23.19 N.

Next, the obtained amorphous compound was subjected to the above-mentioned DSC analysis, XRD analysis, and solution rate measurement.

### (Comparative Example 1)

The compound (1) was subjected to the DSC analysis, XRD analysis, and solution rate measurement carried out in the same manner as described above, except that the "compound (1)" was used as it was in place of the "obtained amorphous compound."

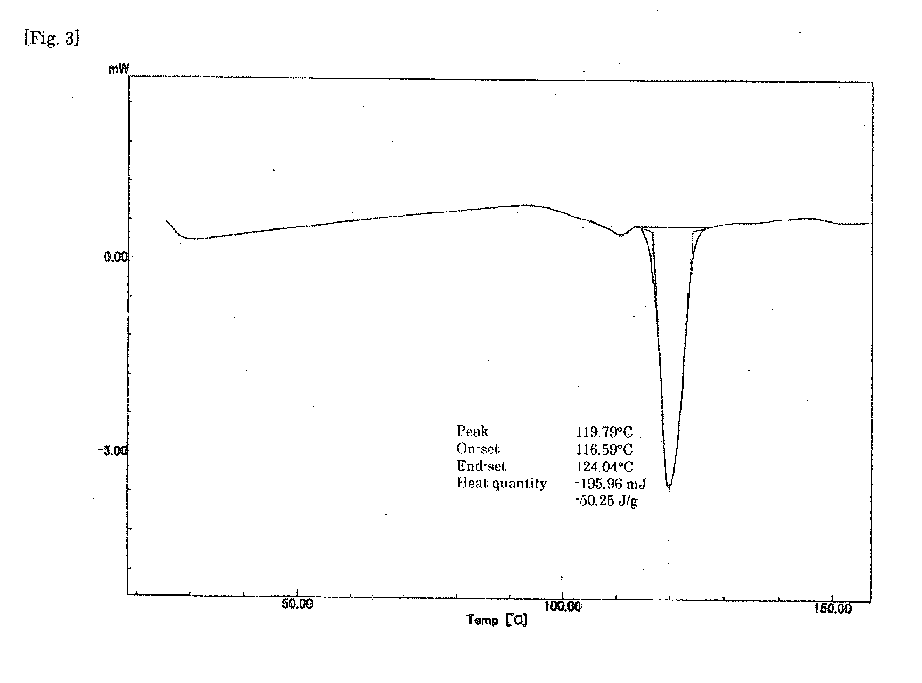

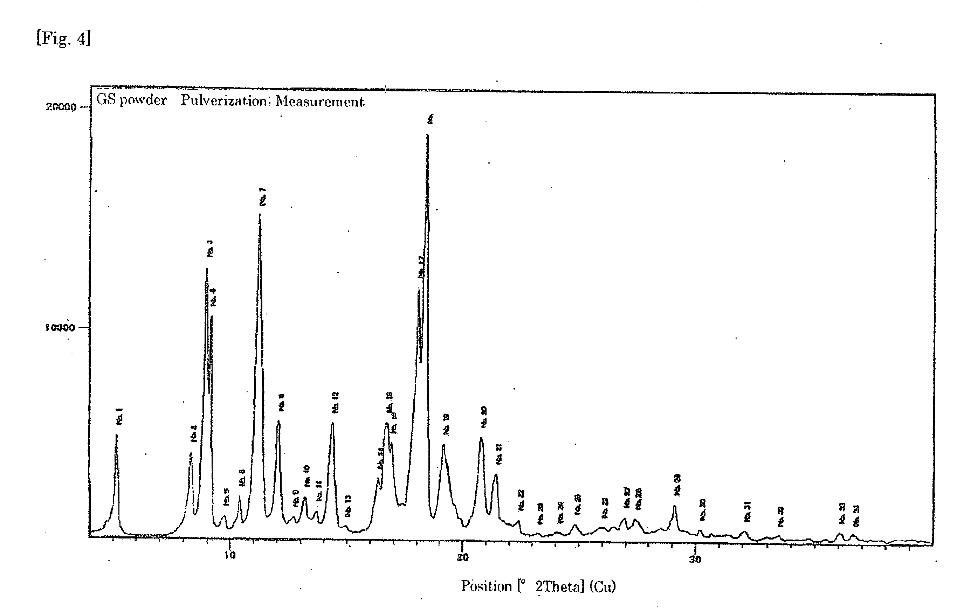

The results are shown in Fig. 3 (DSC analysis), Fig. 4 (XRD analysis), and Table 1 (weight, hardness, particle diameter (width) and height, and solution rate).

**Table 1**

| | Test substance | Weight (mg/particle) | Hardness (N) | Particle diameter/height (mm) | Solution rate (mg/sec) |
|---|---|---|---|---|---|
| Example 1 | amorphous compound | 8,62 | 24.79 | 3.4/2.2 | 7.407 |
| Example 2 | amorphous compound | 9.65 | 23.77 | 3,1/1.8 | 6.579 |
| Example 3 | amorphous compound | 9.99 | 23.19 | 2.7/1.7 | 5.556 |
| Comparative Example 1 | crystalline substance compound (1) | <1.0 | - | fine white powder | 3.367 |

### (Example 4)

Stabilizers for polymers having 100% by weight of the content of the amorphous compounds (approximately semi-spherical amorphous compounds) obtained in Examples 1 to 3 in the entire weight were prepared. The stabilizers for polymers in a ratio of 10 parts by weight were stirred with 100 parts by weight of each of four kinds of hydrocarbon solvents; pentane, hexane, cyclopentane, and cyclohexane; at 25°C and dissolved. During the work, the powder scattering state to be generated from the stabilizers for polymers was observed, but it was confirmed that occurrence of powder scattering was not observed in any of these hydrocarbon solvents. Next, the dissolved materials were added in a ratio proper to adjust the amount of the amorphous compound 0.5 parts by weight to 100 parts by weight of a styrene-butadiene copolymer, as a thermoplastic polymer and the solvent was removed to produce polymer compositions.

### (Comparative Example 2)

A polymer composition was produced in the same manner as in Example 4 by using the stabilizer for polymers obtained as a test substance used in Comparative Example 1. During the work of stirring the stabilizer for polymers in a ratio of 10 parts by weight with 100 parts by weight of four kinds of hydrocarbon solvents; pentane, hexane, cyclopentane, and cyclohexane; at 25°C and dissolving the stabilizer for polymers, the powder scattering state to be generated from the stabilizer for polymers was observed, and it was confirmed that occurrence of powder scattering was observed in any of these hydrocarbon solvents.

**Table 2**

| | Test substance (efficacious component) | Occurrence of powder scattering | Polymer stabilization effect | Remarks |
|---|---|---|---|---|
| Example 4 | Example 1 | none | effective | stabilizer for polymers of the invention |
| Example 5 | Example 2 | none | effective | stabilizer for polymers of the invention |
| Example 6 | Example 3 | none | effective | stabilizer for polymers of the invention |
| Comparative Example 2 | Comparative Example 1 | observed | effective | stabilizer for polymers for comparison |

### (Example 5)

Polymerization of 1,3-butadiene was carried out at 60 to 65°C in a nitrogen atmosphere and cyclohexane using n-butyl lithium as a catalyst.
In this connection, isopropyl alcohol was used as a polymerization
terminator.

Further, the stabilizer for polymers prepared in Example 4 was stirred at a ratio of 20 parts by weight with 100 parts by weight of cyclohexane at 25°C and dissolved.

Next, the obtained dissolved material was blended at a proper ratio to give a ratio of the stabilizer for polymers in the dissolved material to be 1 part by weight to 100 parts by weight of the obtained polymer and cyclohexane in the obtained mixture was flush-evaporated at 190 to 200°C in a nitrogen atmosphere to obtain a polybutadiene rubber composition.

### (Example 6)

After 0.5 parts by weight of the stabilizer for polymers prepared in Example 4 (particulate) and 100 parts by weight of a styrene-butadiene copolymer (MI (200°C, load 5.0 kg): 12 g/10 min, manufactured by BASF) as a thermoplastic polymer were dry-blended, the mixture was kneaded at a screw rotation speed of 100 rpm at 200°C using a uniaxial extruder (VS 30-28 type extruder, manufactured by Tanabe Plastics Co., Ltd.) with 30 mm diameter to obtain pellets of a styrene-butadiene copolymer composition containing the stabilizer for polymers evenly dispersed in the styrene-butadiene copolymer.

The present amorphous compound having excellent "capability of quickly dissolving in a hydrocarbon solvent" is remarkably useful as an efficacious component for a stabilizer for polymers and the stabilizer for polymers of the invention containing the amorphous compound causes no powder scattering and is thus excellent as a stabilizer for polymers.

## Claims

1. An amorphous compound defined by the following formula (1): 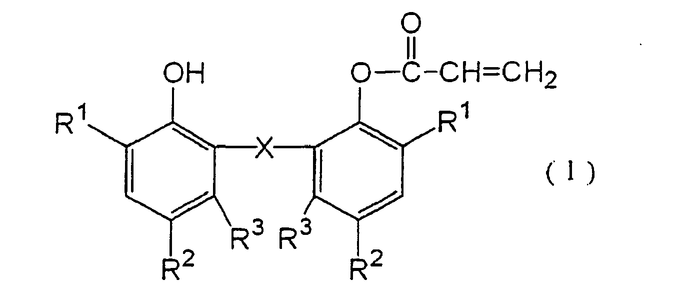 wherein R¹ and R² independently denote a hydrogen atom, an alkyl group
with 1 to 8 carbon atoms, or a cycloalkyl group with 5 to 8 carbon atoms; R³ independently denotes a hydrogen atom or an alkyl group with 1 to 8 carbon atoms; X denotes a single bond, a sulfur atom, an oxygen atom, an alkylidene group with 1 to 8 carbon atoms, or a cycloalkylidene group with 5 to 8 carbon atoms: having an endothermic peak at 15 to 25°C determined by differential scanning calorimetry (DSC) and a solution rate of 5 mg/sec or higher in cyclohexane at 25°C in the following test system:
in the test system for solution rate,
the measurement is carried out by loading a container (capacity: 100 ml, outer diameter: 55 mm, height: 70 mm) containing 50 g of cyclohexane at 25°C with 3 g of a test substance; rotating fan type stirring blades with 38 mm diameter at a rotation speed of 100 rpm; and measuring the time taken for the test substance to be dissolved.

2. The amorphous compound according to claim 1 wherein in the formula (1), R¹ and R² are each a tert-pentyl group; R³ is a hydrogen atom; and X is an ethylidene group.

3. The amorphous compound according to claim 2 being in an
atomic arrangement state shown by the X-ray diffraction pattern of Fig. 2 determined by X-ray diffractometry using a CuKα spectrum.

4. The amorphous compound according to claim 1, wherein in the formula (1), R¹ is a tert-butyl group; R² is a methyl group; R³ is a hydrogen atom; and X is a methylene group.

5. A process for producing the amorphous compound according to any one of claims 1 to 4, comprising a first step of melting a crystalline substance having a melting point of 70°C to 220°C and defined by the following formula (1): 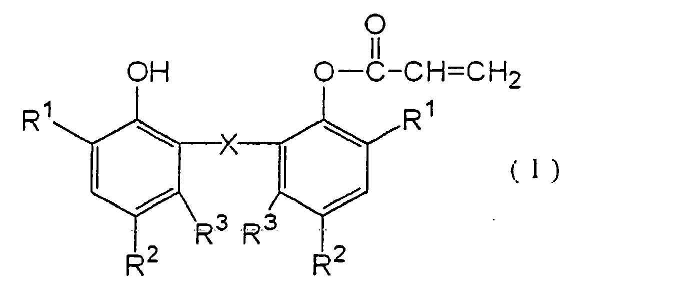 wherein R¹ and R² independently denote a hydrogen atom, an alkyl group with 1 to 8 carbon atoms, or a cycloalkyl group with 5 to 8 carbon atoms; R³ denotes a hydrogen atom or an alkyl group with 1 to 8 carbon atoms; X denotes a single bond, a sulfur atom, an oxygen atom, an alkylidene group with 1 to 8 carbon atoms, or a cycloalkylidene group with 5 to 8 carbon atoms by heating at a temperature equal to or higher than the melting point and a second step of cooling and solidifying the melted substance obtained in the first step.

6. A stabilizer for polymers, containing the amorphous compound according to any of claims 1 to 4.

7. The stabilizer for polymers according to claim 6, having a particulate shape.

8. A process for producing a polymer composition, comprising a first step of dissolving the stabilizer for polymers according to claim 6 or 7 in a hydrocarbon solvent and a second step of blending the dissolved material obtained in the first step with a polymer.

9. The process according to claim 8, wherein the polymer is a thermoplastic polymer.

## Patentansprüche

1. Eine amorphe Verbindung der folgenden Formel (1): 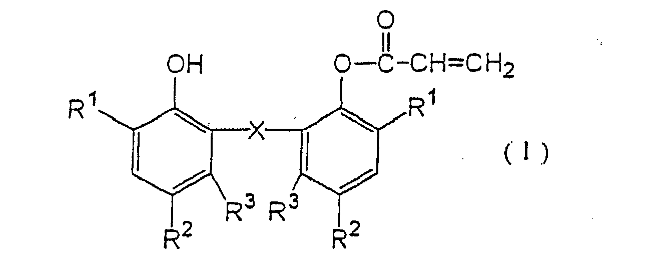 wobei R¹ und R² unabhängig ein Wasserstoffatom, eine Allylgruppe mit 1 bis 8 Kohlenstoffatomen, oder eine Cycloalkylgruppe mit 5 bis 8 Kohlenstoffatomen bezeichnen, R³ unabhängig ein Wasserstoffatom oder eine Alkylgruppe mit 1 bis 8 Kohlenstoffatomen bezeichnet; X eine Einfachbindung, ein Schwefelatom, ein Sauerstoffatom, eine Alkylidengruppe mit 1 bis 8 Kohlenstoffatomen oder eine Cycloalkylidengruppe mit 5 bis 8 Kohlenstoffatomen bezeichnet:
die einen durch Differential Scanning Calorimetry (DSC) gemessenen endothermen Peak bei 15 bis 25°C und eine Lösungsrate von 5 mg/s oder mehr in Cyclohexan bei 25°C in dem folgenden Testsystem aufweist:
im Testsystem für die Lösungsrate wird die Messung durch Beladen eines Behälters (Fassungsvermögen: 100 ml, Außendurchmesser: 55 mm, Höhe: 70 mm), der 50 g Cyclohexan bei 25°C enthält, mit 3 g einer Testsubstanz durchgeführt; Rührblätter vom Typ eines rotierenden Lüfters mit 38 mm Durchmesser bei einer Drehgeschwindigkeit von 100 Umdrehungen pro Minute; und Messen der Zeit, die es zur Auflösung der Testsubstanz braucht.

2. Die amorphe Verbindung gemäß Anspruch 1, wobei in der Formel (1) R¹ und R² jeweils eine tert-Pentylgruppe sind, R³ ein Wasserstoffatom ist und X eine Ethylidengruppe ist.

3. Die amorphe Verbindung gemäß Anspruch 2, die sich in einem Zustand atomarer Anordnung befindet, der durch das Röntgen-Beugungsmuster von Abbildung 2 beschrieben ist, welche durch Röntgenbeugung unter Verwendung eines CuKα-Spektrums bestimmt wird.

4. Die amorphe Verbindung gemäß Anspruch 1, wobei in der Formel (1) R¹ eine *tert-*Butylgruppe ist, R² eine Methylgruppe ist, R³ ein Wasserstoffatom ist, und X eine Methylengruppe ist.

5. Ein Verfahren zur Herstellung der amorphen Verbindung gemäß einem der Ansprüche 1 bis 4, umfassend einen ersten Schritt des Schmelzens einer kristallinen Substanz mit einem Schmelzpunkt von 70°C bis 220°C und der folgenden Formel (1): 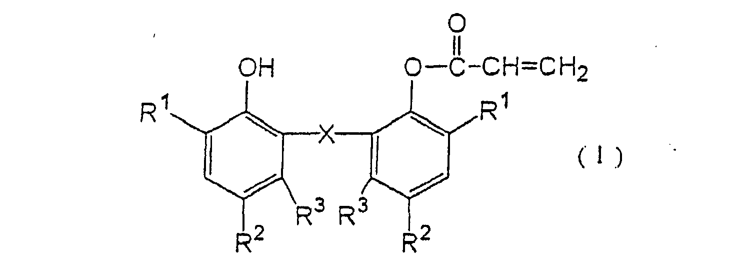 wobei R¹ und R² unabhängig ein Wasserstoffatom, eine Alkylgruppe mit 1 bis 8 Kohlenstoffatomen, oder eine Cycloalkylgruppe mit 5 bis 8 Kohlenstoffatomen bezeichnen, R³ ein Wasserstoffatom oder eine Alkylgruppe mit 1 bis 8 Kohlenstoffatomen bezeichnet; X eine Einfachbindung, ein Schwefelatom, ein Sauerstoffatom, eine Alkylidengruppe mit 1 bis 8 Kohlenstoffatomen oder eine Cycloalkylidengruppe mit 5 bis 8 Kohlenstoffatomen bezeichnet,
durch Erhitzen auf eine Temperatur höher oder gleich dem Schmelzpunkt und einen zweiten Schritt des Kühlens und Verfestigens der im ersten Schritt erhaltenen geschmolzenen Substanz.

6. Ein Stabilisator für Polymere, enthaltend die amorphe Verbindung gemäß einem der Ansprüche 1 bis 4.

7. Der Stabilisator für Polymere gemäß Anspruch 6 mit einer teilchenförmigen Form.

8. Verfahren zur Herstellung einer Polymerzusammensetzung, umfassend einen ersten Schritt des Auflösens des Stabilisators für Polymere gemäß Anspruch 6 oder 7 in einem Kohlenwasserstofflösungsmittel und einen zweiten Schritt des Mischens des im ersten Schritt erhaltenen gelösten Stoffs mit einem Polymer.

9. Das Verfahren gemäß Anspruch 8, wobei das Polymer ein thermoplastisches Polymer ist.

## Revendications

1. Composé amorphe défini par la formule suivante (1) : 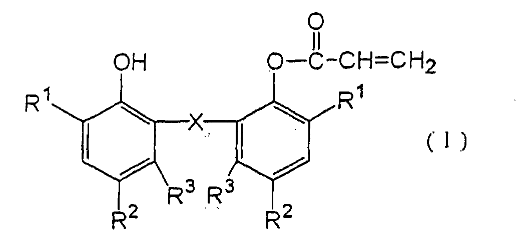 dans laquelle R¹ et R² désignent indépendamment un atome d'hydrogène, un groupe alkyle avec 1 à 8 atomes de carbone, ou un groupe cycloalkyle avec 5 à 8 atomes de carbone ; R³ désigne indépendamment un atome d'hydrogène ou un groupe alkyle avec 1 à 8 atomes de carbone ; X désigne une liaison simple, un atome de soufre, un atome d'oxygène, un groupe alkylidène avec 1 à 8 atomes de carbone, ou un groupe cycloalkylidène avec 5 à 8 atomes de carbone ayant un pic endothermique à 15 à 25 °C déterminé par une calorimétrie à balayage différentiel (DSC) et un débit de solution de 5 mg/s ou plus dans du cyclohexane à 25 °C dans le système de test suivant :
dans le système de test pour le débit de solution,
la mesure est effectuée en chargeant un récipient (capacité : 100 ml, diamètre extérieur : 55 mm, hauteur : 70 mm) contenant 50 g de cyclohexane à 25 °C avec 3 g d'une substance de test ; pales d'agitation de type ventilateur rotatif de 38 mm de diamètre à une vitesse de rotation de 100 tr/min ; et mesure du temps mis pour dissoudre la substance de test.

2. Composé amorphe selon la revendication 1, dans lequel, dans la formule (1), R¹ et R² sont chacun un groupe tert-pentyle ; R³ est un atome d'hydrogène ; et X est un groupe éthylidène.

3. Composé amorphe selon la revendication 2, étant dans un état d'agencement atomique représenté par le motif de diffraction aux rayons X de la figure 2 déterminé par une diffractométrie aux rayons X en utilisant un spectre CuKα.

4. Composé amorphe selon la revendication 1, dans lequel dans la formule (1), R¹ est un groupe tert-butyle ; R² est un groupe méthyle ; R³ est un atome d'hydrogène ; et X est un groupe méthylène.

5. Processus pour produire le composé amorphe selon l'une quelconque des revendications 1 à 4, comprenant une première étape de fusion d'une substance cristalline ayant un point de fusion de 70 °C à 220 °C et défini par la formule suivante (1) : 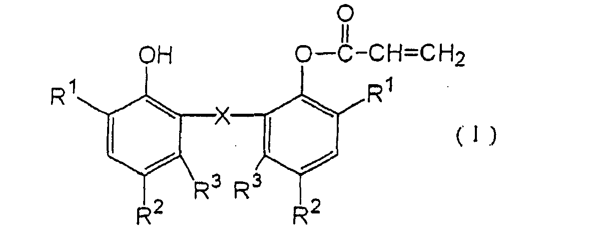 dans laquelle R¹ et R² désignent indépendamment un atome d'hydrogène, un groupe alkyle avec 1 à 8 atomes de carbone, ou un groupe cycloalkyle avec 5 à 8 atomes de carbone ; R³ désigne un atome d'hydrogène ou un groupe alkyle avec 1 à 8 atomes de carbone ; X désigne une liaison simple, un atome de soufre, un atome d'oxygène, un groupe alkylidène avec 1 à 8 atomes de carbone, ou un groupe cycloalkylidène avec 5 à 8 atomes de carbone par chauffage à une température égale ou supérieure au point de fusion et une seconde étape de refroidissement et de solidification de la substance fondue obtenue dans la première étape.

6. Stabilisateur pour polymères, contenant le composé amorphe selon l'une quelconque des revendications 1 à 4.

7. Stabilisateur pour polymères selon la revendication 6, ayant une forme particulairé.

8. Processus pour produire une composition de polymère, comprenant une première étape de dissolution du stabilisateur pour polymères selon la revendication 6 ou 7 dans un solvant d'hydrocarbure et une seconde étape de mélange de la matière dissoute obtenue dans la première étape avec un polymère.

9. Processus selon la revendication 8, dans lequel le polymère est un polymère thermoplastique.
